# EUROPÄISCHE PATENTSCHRIFT

(11) **EP 3 884 893 B1**
(45) Veröffentlichungstag und Bekanntmachung des Hinweises auf die Patenterteilung: **28.12.2022**
(21) Anmeldenummer: 21163547.9
(22) Anmeldetag: 18.03.2021
(51) Int. Cl.: A61B 18/10, H02J 7/00, H02M 1/32

(54) **MEDIZINGERÄT MIT EINEM BETRIEBSSICHEREN NETZTEIL**
MEDICAL DEVICE WITH A RELIABLE POWER SUPPLY
APPAREIL MÉDICAL DOTÉ D'UN BLOC SECTEUR FIABLE

(30) Priorität: 19.03.2020 DE 102020107614
(43) Veröffentlichungstag der Anmeldung: 29.09.2021
(73) Patentinhaber: Olympus Winter & Ibe GmbH, 22045 Hamburg (DE)
(72) Erfinder: Prezewowsky, Thomas, 14513 Teltow (DE)
(74) Vertreter: Eisenführ Speiser

(56) Entgegenhaltungen:
- DE-A1-102009 037 693
- US-A- 5 523 665
- US-B2- 6 703 793

## Beschreibung

Die Erfindung betrifft ein Medizingerät mit einem Netzteil zum Anschluss an ein Wechselstromnetz, wobei das Netzteil einen Gleichrichter und wenigstens einen Siebkondensator zum Glätten einer von dem Netzteil auszugebenden Gleichspannung sowie wenigstens einen Entladewiderstand zum Entladen des Siebkondensators aufweist. Die Erfindung betrifft insbesondere einen Elektrochirurgie-Generator mit Ausgängen, an die ein elektrochirurgisches Instrument angeschlossen und über die ein angeschlossenes elektrochirurgisches Instrument mit einer hochfrequenten Ausgangswechselspannung versorgt werden kann.

Aus Sicherheitsgründen soll an Kondensatoren eines elektrischen Geräts in angemessener Zeit nach dessen Ausschalten keine höhere Spannung mehr anliegen. Dies betrifft auch Medizingeräte und insbesondere Hochspannungsgleichstromnetzteile von Elektrochirurgie-Generatoren. Zu diesem Zweck sind Entladewiderstände vorgesehen, die parallel zu hochkapazitiven Kondensatoren liegen und diese entladen.

Aus dem US-Patent 5,523,665 A ist ein Netzteil mit einer Gleichrichterbrücke und Siebkondensatoren bekannt, die nach dem Wegfall der Netzspannung über einen Widerstand entladen werden.

Der Erfindung liegt die Aufgabe zugrunde, ein verbessertes Netzteil insbesondere für ein Medizingerät wie einen Elektrochirurgie-Generator zu schaffen, welches die Sicherheitsanforderungen erfüllt.

Erfindungsgemäß wird diese Aufgabe durch ein Medizingerät mit einem Netzteil zum Anschluss an ein Wechselstromnetz gelöst, das eine Schaltung aufweist, um zumindest einen Entladewiderstand nur dann parallel zu wenigstens einem Sieb-Kondensator zu schalten, wenn keine Eingangswechselspannung an dem Netzteil anliegt.

Das Netzteil weist einen Gleichrichter und wenigstens einen Siebkondensator zum Glätten einer von dem Netzteil auszugebenden Gleichspannung sowie wenigstens einen Entladewiderstand zum Entladen des Siebkondensators auf. Außerdem weist das Netzteil eine Schaltung auf, die mit dem Entladewiderstand und dem Siebkondensator elektrisch verbunden und die ausgebildet ist, einen eingeschalteten Zustand des medizinischen Gerätes, bei dem eine Eingangswechselspannung an dem Netzteil anliegt, zu detektieren und den Entladewiderstand nur dann parallel zu dem Siebkondensator zu schalten, wenn die Schaltung keinen eingeschalteten Zustand des medizinischen Gerätes detektiert.

Die Schaltung weist einen ersten Transistor (T2) zum Parallelschalten des Entladewiderstands (R4, R5, R6, R7, R8, R9) zu dem Siebkondensator (C1) auf. Der erste Transistor (T2) ist so angeordnet und dimensioniert, dass er durchschaltet und ein Entladen des Siebkondensators (C1) bewirkt, wenn ein mit der Basis oder dem Gate des ersten Transistors (T2) verbundener, als Steuerkondensator dienender Kondensator (C4) ausreichend geladen ist. Der Kondensator (C4) wird mittels eines zweiten Transistors (T1), der als Steuertransistor dient, regelmäßig entladen, solange das Netzteil mit Netzspannung versorgt wird.

Die Detektion der Eingangswechselspannung kann somit über den gesamten Eingangsspannungsbereich des Medizingerätes sicher erfolgen und die zusätzliche notwendige Energie zur Versorgung der Detektionsschaltung ist minimiert.

Vorzugsweise weist die Schaltung einen Schalter zum Parallelschalten des Entladewiderstands zu dem Siebkondensator auf. Der Schalter ist vorzugsweise ein Feldeffekttransistor.

Vorgeschlagen wird somit ein Netzteil mit einer elektronischen Schaltung, die einen oder mehrere Entladewiderstände erst dann parallel zu einem oder mehreren entsprechenden Kondensatoren des Netzteils - insbesondere Siebkondensatoren - schaltet, wenn das Medizingerät ausgeschaltet wird. Dazu wird die Versorgungsspannung (Wechselspannung) detektiert und als Steuergröße verwendet.

Die Erfindung schließt die Erkenntnis ein, dass je nach Spannungswert und Kapazität der Sieb-Kondensatoren, über diese Entladewiderstände mehr oder weniger hohe Ströme fließen, die im eingeschalteten Zustand eine permanente Verlustleistung erzeugen, und damit die Stromaufnahme z. B. eines Elektrochirurgie-Generators im Standby-Betrieb deutlich erhöht. Dies erhöht die Innentemperatur des Medizinproduktes und vermindert die mittlere Betriebsdauer zwischen Ausfällen des Medizingeräts.

Vorzugsweise weist der Gleichrichter eine Gleichrichterbrücke auf, und die Schaltung ist vorzugsweise dazu konfiguriert, eine Spannung zwischen einem eingangsseitigen Anschluss der Gleichrichterbrücke und einem ausgangsseitigen Anschluss der Gleichrichterbrücke abzugreifen und eine an dem eingangsseitigen Anschluss der Gleichrichterbrücke anliegende Versorgungsnetzspannung zu detektieren.

Hierzu weist die Schaltung vorzugsweise einen zwischen einem Eingang der Gleichrichterbrücke und dem negativen Ausgang der Gleichrichterbrücke geschalteten Spannungsteiler und einen einem Widerstand des Spannungsteilers parallel geschalteten ersten Kondensator auf, der Im Falle eines Anliegens einer Versorgungsnetzspannung netzseitig eventuell auftretende Störimpulse filtert und für das notwendige Teilungsverhältnis des Spannungsteilers sorgt.

Weiterhin ist die Basis-Emitter-Strecke des Steuertransistors vorzugsweise dem Widerstand des Spannungsteilers parallel geschaltet und der Steuertransistor schaltet im Falle eines Anliegens einer Versorgungsnetzspannung im Bereich von >50 V_{AC} ... 264 V_{AC} im Takt der Eingangswechselspannung durch. Der Steuertransistor wird somit vorzugsweise in einer Emitterschaltung betrieben. Der Steuertransistor ist vorzugsweise ein Bipolartransistor, insbesondere ein npn-Transistor. Der Steuertransistor kann aber auch ein pnp Transistor sein und die Schaltung entsprechend komplementär aufgebaut sein. Eine entsprechende Schaltung mit einem Feldeffekttransistor ist alternativ ebenso möglich.

Vorzugsweise ist ein zweiter Kondensator einer Gate-Drain-Strecke des als Schalter dienenden Feldeffekttransistors parallel geschaltet. Dieser zweite Kondensator bewirkt in seinem geladenen Zustand, dass der Feldeffekttransistor durchschaltet, so dass der wenigstens eine Entladewiderstand zu dem wenigstens einen Siebkondensator parallel geschaltet ist. Der zweite Kondensator wird aus dem Siebkondensator geladen, wenn keine Versorgungsnetzspannung an dem Gleichrichter bzw. dessen Gleichrichterbrücke anliegt und bewirkt, dass der als Schalter dienende Feldeffekttransistor durchschaltet und somit der wenigstens eine Entladewiderstand dem wenigstens einen Siebkondensator parallel geschaltet ist. Damit wird der Siebkondensator über den Entladewiderstand entladen, wenn an einem Eingang des Gleichrichters keine Versorgungsnetzspannung anliegt.

Der zweite Kondensator ist vorzugsweise einer Kollektor-Emitter-Strecke des Steuertransistors parallel geschaltet, so dass der zweite Kondensator entladen wird, wenn der Steuertransistor durchgeschaltet ist. Wenn der zweite Kondensator entladen ist, sperrt der als Schalter dienende Feldeffekttransistor so dass der wenigstens eine Entladewiderstand vom dem wenigstens einen Siebkondensator getrennt ist. Der Steuertransistor schaltet durch, wenn an dem Eingang des Gleichrichters eine Versorgungsnetzspannung anliegt und bewirkt somit, dass der Entladewiderstand von dem Siebkondensator bei anliegender Versorgungsnetzspannung getrennt ist.

Vorzugsweise weist die Schaltung ein oder mehrere Zener-Dioden zur Spannungsbegrenzung auf. Eine jeweilige Zener-Diode ist insbesondre zum Schutz eines jeweiligen Transistors vorgesehen.

Vorzugsweise weist die Schaltung mehrere, einander parallel geschaltete Entladewiderstände auf, um auf diese Weise auch eine größere Verlustleistung beim Entladen des Siebkondensators zu ermöglichen.

Der wenigstens eine Entladewiderstand hat vorzugsweise einen Widerstandswert zwischen 47 kΩ und 120 kΩ. Allgemein ist der Widerstandwert des Entladewiderstands vorzugsweise so zu wählen, dass 60 Sekunden nach dem Abschalten der maximalen Eingangswechselspannung die Gleichspannung an dem wenigstens einen Siebkondensator auf weniger als 60 V abgesunken ist. Der bevorzugte Widerstandwert des wenigstens einen Entladewiderstands ergibt sich somit aus der maximalen Eingangswechselspannung und der Kapazität des wenigstens einen Siebkondensators.

Die Erfindung soll nun anhand eines Ausführungsbeispiels mit Bezug auf die Figuren näher erläutert werden. Von den Figuren zeigt
- Fig. 1:: eine schematische Darstellung einiger Komponenten eines Elektrochirurgie-Generators zur Versorgung eines elektrochirurgischen Instruments mit einer hochfrequenten Wechselspannung;
- Fig. 2:: eine Schaltung, die ein erfindungsgemäßes Parallelschalten von wenigstens einem Entladewiderstand zu wenigstens einem Siebkondensator eines Gleichspannungsnetzteils nach dessen Trennen von einer Netzversorgungs-Wechselspannung ermöglicht; und
- Fig. 3: eine Schaltung ähnlich der in Figur 2 abgebildeten Schaltung, allerdings für ein Netzteil, das eine symmetrische Ausgangsspannung liefert.

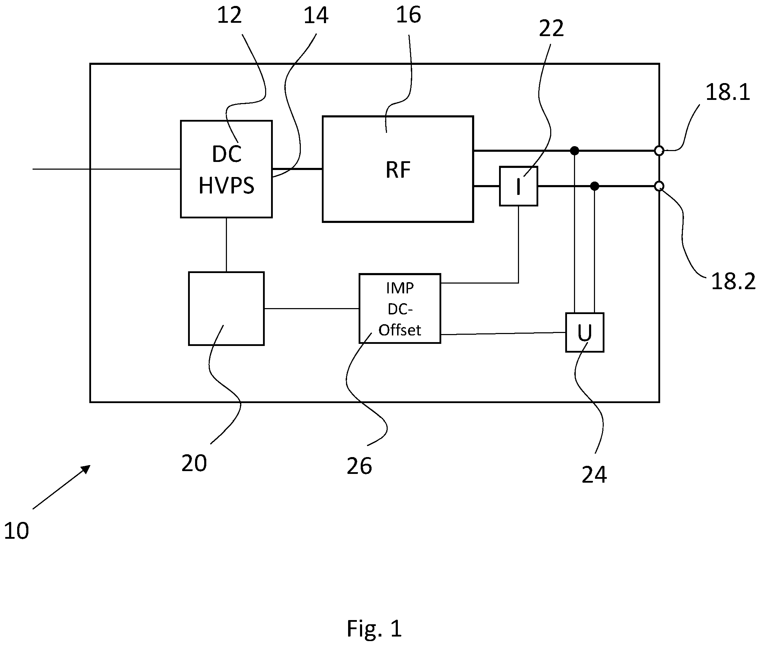

Figur 1 zeigt eine Medizingerät am Beispiel eines Elektrochirurgie-Generators 10. Wie Figur 1 zu entnehmen ist, weist der Elektrochirurgie-Generator 10 hierzu ein Hochspannungsnetzteil 12 (HVPS; High Voltage Power Supply) auf, das beispielsweise an das übliche öffentliche Stromversorgungsnetz angeschlossen werden kann und an seinem Ausgang 14 einen Hochspannungsgleichstrom bereitstellt. Dieser Hochspannungsgleichstrom wird einem Hochfrequenzteil 16 des Elektrochirurgie-Generators 10 zugeführt. Der Hochfrequenzteil 16 des Elektrochirurgie-Generators 10 wirkt als Wechselrichter und erzeugt eine hochfrequente Wechselspannung, die über einen Ausgangstransformator (nicht dargestellt) des Hochfrequenzteils 16 an Ausgänge 18.1 und 18.2 des Elektrochirurgie-Generators 10 abgegeben wird. An die Ausgänge 18.1 und 18.2 des Elektrochirurgie-Generators 10 kann ein elektrochirurgisches Instrument angeschlossen werden. Die Ausgangsleistung des Elektrochirurgie-Generators 10 kann mittels einer Steuereinheit 20 und Erfassungseinheiten 22 und 24 sowie einer Auswerteinheit 26 zum Bilden von aus erfassten Werten abgeleiteten Werten wie Impedanz etc. gesteuert oder geregelt werden.

Aktuelle Hochfrequenz Elektrochirurgie-Generatoren erzeugen die HF-Ausgangsspannung in zwei Schritten. Zunächst wird die Netzeingangsspannung in eine variable Gleichspannung umgesetzt. Diese dient als Eingangsspannung für eine Wechselrichterschaltung des Hochspannungsnetzteils 12, dessen Ausgangsspannung proportional zur Eingangsspannung zunimmt. Die Ausgangsspannung (und damit auch Strom und Leistung) kann daher mit Hilfe der Eingangsspannung geregelt werden.

Für das Hochspannungsnetzteil 12 gilt, dass es gemäß IEC60601-1 in der gültigen Fassung, notwendig ist Vorkehrungen zu treffen, dass an Energiespeichern (meist Kondensatoren) eine Minute nach Ausschalten eines elektronischen Medizinproduktes eine Spannung <60 V anliegt.

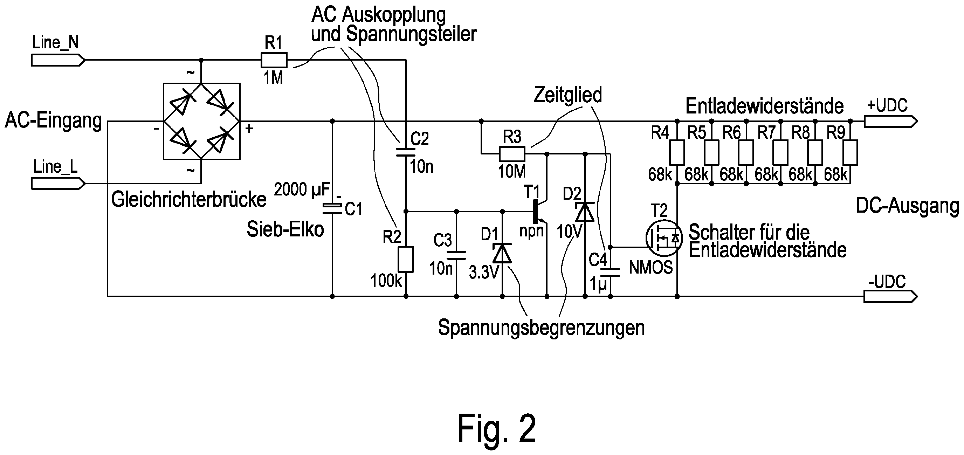

Figur 2 zeigt ein Beispiel für ein erfindungsgemäßes Hochspannungsnetzteil.

Dies weist eine elektronische Schaltung auf, die die notwendigen Entladewiderstände erst dann parallel zu den entsprechenden Kondensatoren schaltet, wenn das elektronische Medizingerät ausgeschaltet wird. Dazu wird die Versorgungsspannung (Wechselspannung) detektiert und als Steuergröße verwendet. Voraussetzung ist, dass das Gerät mit einer Versorgungsspannung betrieben wird, die zwischen 100 und 240 VAC liegt.

Ein Netzteil zum Erzeugen einer Ausgangsgleichspannung aus einer Eingangswechselspannung weist typischerweise eine von vier Dioden gebildete Gleichrichterbrücke zum Gleichrichten des Wechselstroms und eine oder mehrere Sieb-Kondensatoren zum Glätten der Ausgangsgleichspannung auf. Die Gleichrichterbrücke weist zwei Eingänge für die Eingangswechselspannung und zwei Ausgänge für die Ausgangsgleichspannung auf. Ein Ausgang ist positiv, der andere Ausgang der Gleichrichterbrücke ist negativ gepolt.

Nach Abschalten der Versorgungsspannung - also des Eingangswechselstroms - sind die Sieb-Kondensatoren zunächst noch geladen. In dem in Figur 2 dargestellten Ausführungsbeispiel ist ein Sieb-Kondensator C1 in Form eine Elektrolyt-Kondensator mit einer Kapazität von 2000 µF vorgesehen. Anstelle eines einzigen Siebkondensators können auch mehrere zueinander parallel oder in Serie geschaltete Siebkondensatoren vorgesehen sein. Wenn die Ausgangsgleichspannung des Hochspannungsnetzteils beispielsweise 300 V beträgt, können die Siebkondensatoren paarweise in Serie geschaltet sein, so dass über jeden der Siebkondensatoren nur eine maximale Spannung von 150 V abfällt. Durch Parallelschalten der Siebkondensatoren kann die Siebkapazität erhöht werden.

Gemäß dem Ausführungsbeispiel in Figur 2 sind insgesamt 6 Entladewiderstände R4 bis R9 vorgesehen, die einander parallel geschaltet sind, um insgesamt höher belastbar zu sein. Jeder der Widerstände R4 bis R9 hat einen Widerstandswert von 68 kΩ.

Um den Sieb-Kondensator C1 nach Abschalten der Versorgungsspannung zu entladen, werden die 6 Entladewiderstände R4 bis R9 dem Sieb-Kondensator C1 parallel geschaltet. Die Anzahl und Dimensionierung der Entladewiderstände ist allgemein in Relation zu der Kapazität der Siebkondensatoren und der maximalen Spannung vorzugsweise so gewählt, dass die Entladewiderstände eine Entladeleistung erlauben, die für den Abbau der maximalen Spannung an den Kondensatoren auf unter 60 V in höchstens 60 Sekunden erforderlich ist.

Damit die 6 Entladewiderstände R4 bis R9 nicht auch bei eingeschaltetem Medizingerät permanent Leistung abführen, sind sie bei eingeschaltetem Medizingerät von dem Sieb-Kondensator C1 getrennt. Hierzu dient ein Feldeffekttransistor T2, der als Schalter dient.

Der Feldeffekttransistor T2 wird von einer Detektionsschaltung angesteuert, die dazu ausgebildet ist, das Anliegen einer Eingangswechselspannung zu detektieren und den Feldeffekttransistor nur dann durchzuschalten - und damit die 6 Entladewiderstände R4 bis R9 dem Sieb-Kondensator C1 parallel zu schalten - wenn keine Eingangswechselspannung an dem Netzteil des Medizingerätes anliegt.

Die Detektionsschaltung weist zur Detektion der Versorgungsspannung und zum Ansteuern des Feldeffekttransistors T2 Widerstände R1 (1 MΩ) und R2 (100 kΩ) sowie eine Kondensator C2 (10nF) auf, die einander zwischen einem Eingang der Gleichrichterbrücke und dem negativen Ausgang der Gleichrichterbrücke in Serie geschaltet sind.

Die Widerstände R1 (1 MΩ) und R2 (100 kΩ) bilden zusammen mit dem Kondensator C2 (10nF) einen Spannungsteiler. Dem Widerstand R2 ist ein Kondensator C3 (10 nF) parallel geschaltet. Zwischen dem Kondensator C2 und dem Widerstand R2 wird die Spannung abgegriffen und der Basis eines npn-Transistors T1 zugeführt. Der Transistor T1 ist somit ein Bipolartransistor, der in einer Emitterschaltung betrieben wird und als Steuertransistor dient, der im durchgeschalteten Zustand ein Entladen eines Kondensators C4 bewirkt. Der Kondensator C4 ist dem Siebkondensator C1 parallel geschaltet und wird durch den Sieb-kondensator C1 über einen mit dem Kondensator C4 in Serie geschalteten Widertand R3 geladen, falls der Transistor T1 nicht durchgeschaltet ist.

Über den Widerstand R1 und den Kondensator C2 wird die Wechselspannung vor der Gleichrichtung ausgekoppelt und mittels des Widerstands R2 und des Kondensators C3 gefiltert und so verringert, dass sie von dem Transistor T1 (einem npn-Transistor) verarbeitet werden kann. Solange eine Wechselspannung anliegt, wird der Transistor T1 im Takt der Wechselspannung durchgeschaltet und verhindert damit, dass sich der Kondensator C4 über den Widerstand R3 aufladen kann. Dadurch bleibt der Transistor T2 gesperrt und die Entladewiderstände sind stromlos.

Zwei Zener-Dioden D1 (3,3 V) und D2 (10 V) bewirken jeweils eine Spannungsbegrenzung. Die Zenerdiode D1 ist dem Widerstand R2, dem Kondensator C3 und der Basis-Emitter-Strecke des Transtors T1 parallel geschaltet. Die Zener-Dide D2 und ein Kondensator C4 (1µF) sind der Kollektor-Emitter-Strecke des Transistors T1 parallel geschaltet. Solange der Transistor T1 durchgeschaltet ist, fällt über den Kondensator C4 keine Spannung ab, so dass dieser nicht geladen wird. Wenn der Transistor T1 sperrt, wird der Kondensator über den Widerstand R3 geladen. Der Widerstand R3 und der Kondensator C4 sind hierzu zwischen den beiden Ausgängen der Gleichrichterbrücke und parallel zu dem Siebkondensator C1 in Serie zueinander geschaltet. Der Widerstand R3 bestimmt, wie schnell der Kondensator C4 geladen wird und dient somit als Zeitglied. Die Zener-Diode D2 begrenzt die Spannung über den Kondensator C4 auf maximal 10 Volt. Der Kondensator C4 kann somit auf maximal 10 Volt geladen werden.

Über die Spannung über den Kondensator C4 wird das Gate des Feldeffekttransistors T2 angesteuert.

Wird die Wechselspannung am Eingang der Gleichrichterbrücke abgeschaltet, so sperrt der Transistor T1 und der Kondensator C4 (1µF) kann sich über den Widerstand R3 bis auf 10 V aufladen. Damit wird der Feldeffekttransistor T2 leitend und die Entladewiderstände R4 bis R9 entladen den Sieb-Kondensator C1. Bei Wiederkehr der Wechselspannung wird der Kondensator C4 über den Transistor T1 entladen und der Feldeffekttransistor T2 sperrt. Damit sind die Entladewiderstände R4 bis R9 wieder stromlos.

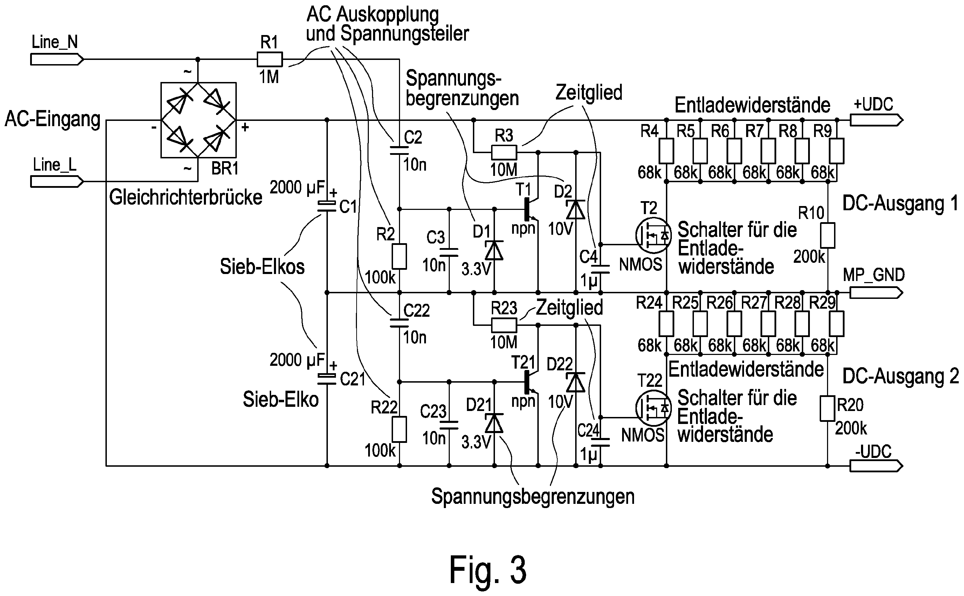

Figur 3 zeigt eine Schaltung für ein Netzteil zum Erzeugen einer symmetrischen Ausgangsgleichspannung. Die Schaltung ist weitestgehend eine Verdoppelung der in Figur 2 abgebildeten Schaltung. Die beiden zusätzlichen Widerstände R10 und R20 dienen zur Symmetrierung der Gleichspannung an den Sieb-Elkos. Wenn die Gleichspannung zwischen positiver Ausgangsgleichspannung +UDC und negativer Ausgangsgleichspannung -UDC gegen Masse MP_GND nicht belastet wird, könnten sich durch unterschiedlich hohe Leckströme in den Kondensatoren, unsymmetrisch hohe Spannungen gegenüber Masse MP_GND einstellen, wenn die AC-Versorgungsspannung vorhanden ist und die Entladewiderstände nicht eingeschaltet sind. R10 und R20 sind vorzugsweise so bemessen, dass sie ca. den fünffachen Leckstrom der Kondensatoren tragen,

Die Schaltung ist für eine Versorgungsspannung (Wechselspannung am Eingang der Gleichrichterbrücke) ausgelegt, die zwischen 100 und 240 VAC liegt.

## Patentansprüche

1. Medizingerät mit einem Netzteil zum Anschluss an ein Wechselstromnetz, wobei das Netzteil einen Gleichrichter und wenigstens einen Siebkondensator (C1) zum Glätten einer von dem Netzteil auszugebenden Gleichspannung sowie wenigstens einen Entladewiderstand (R4, R5, R6, R7, R8, R9) zum Entladen des Siebkondensators (C1) aufweist,
wobei das Netzteil eine Schaltung aufweist, die mit dem Entladewiderstand (R4, R5, R6, R7, R8, R9) und dem Siebkondensator (C1) elektrisch verbunden und die ausgebildet ist, einen eingeschalteten Zustand des medizinischen Gerätes, bei dem eine Eingangswechselspannung an dem Netzteil anliegt, über den gesamten Eingangsspannungsbereich zu detektieren und den Entladewiderstand (R4, R5, R6, R7, R8, R9) nur dann parallel zu dem Siebkondensator (C1) zu schalten, wenn die Schaltung keinen eingeschalteten Zustand des medizinischen Gerätes detektiert, wobei die Schaltung einen ersten Transistor (T2) zum Parallelschalten des Entladewiderstands (R4, R5, R6, R7, R8, R9) zu dem Siebkondensator (C1) aufweist, der so angeordnet und dimensioniert ist, dass er durchschaltet und ein Entladen des Siebkondensators (C1) bewirkt, wenn ein mit der Basis oder dem Gate des Transistors (T2) verbundener Steuerkondensator (C4) ausreichend geladen ist, wobei der Steuerkondensator (C4) mit einem Steuertransistor (T1) verbunden ist, der so angeordnet und dimensioniert ist, dass der Steuertransistor (T1) regelmäßig durchschaltet und den Steuerkondensator (C4) entlädt, solange das Netzteil mit Netzspannung versorgt wird.

2. Medizingerät nach Anspruch 1, wobei der erste Transistor (T2) ein Feldeffekttransistor (T2) ist.

3. Medizingerät nach wenigstens einem der Ansprüche 1 oder 2, wobei der Gleichrichter eine Gleichrichterbrücke aufweist und die Schaltung dazu konfiguriert ist, eine Spannung zwischen einem eingangsseitigen Anschluss der Gleichrichterbrücke und einem ausgangsseitigen Anschluss der Gleichrichterbrücke abzugreifen und eine an dem eingangsseitigen Anschluss der Gleichrichterbrücke anliegende Versorgungsnetzspannung zu detektieren.

4. Medizingerät nach Anspruch 3, wobei die Schaltung einen zwischen einem Eingang der Gleichrichterbrücke und dem negativen Ausgang der Gleichrichterbrücke geschalteten Spannungsteiler und einen einem Widerstand (R2) des Spannungsteilers parallel geschalteten ersten Kondensator (C3) aufweist, der Im Falle eines Anliegens einer Versorgungsnetzspannung auf eine durch den Spannungsteiler definierte Spannung geladen wird.

5. Medizingerät nach wenigstens einem der Ansprüche 1 bis 4, wobei die Basis-Emitter-Strecke des Steuertransistors (T1) dem Widerstand des Spannungsteilers parallel geschaltet ist und der Steuertransistor (T1) im Falle eines Anliegens einer Versorgungsnetzspannung im Bereich von >50 V_{AC} ... 264 V_{AC} durchschaltet.

6. Medizingerät nach Anspruch wenigstens einem der Ansprüche 1 bis 5, wobei der Steuertransistor (T1) ein Bipolartransistor ist.

7. Medizingerät nach wenigstens einem der Ansprüche 2 bis 6, wobei einer Gate-Drain-Strecke des Feldeffekttransistors (T2) ein zweiter Kondensator (C4) parallel geschaltet ist, der in seinem geladenen Zustand bewirkt, dass der Feldeffekttransistor (T2) durchschaltet, so dass der wenigstens eine Entladewiderstand (R4, R5, R6, R7, R8, R9) zu dem wenigstens einen Siebkondensator (C1) parallel geschaltet ist.

8. Medizingerät gemäß der Ansprüche 6 und 7, wobei der zweite Kondensator (C4) einer Kollektor-Emitter-Strecke des Steuertransistors (T1) parallelgeschaltet ist, so dass der zweite Kondensator (C4) entladen wird, wenn der Steuertransistor (T1) durchgeschaltet ist, so dass der wenigstens eine Entladewiderstand (R4, R5, R6, R7, R8, R9) vom dem wenigstens einen Siebkondensator (C1) getrennt ist, wenn an dem Eingang des Gleichrichters eine Versorgungsnetzspannung anliegt.

9. Medizingerät nach wenigstens einem der Ansprüche 1 bis 8, wobei die Schaltung wenigstens eine Zener-Diode (D1; D2) zur Spannungsbegrenzung aufweist.

10. Medizingerät nach wenigstens einem der Ansprüche 1 bis 9, wobei die Schaltung mehrere, einander parallel geschaltete Entladewiderstände (R4, R5, R6, R7, R8, R9) aufweist.

11. Medizingerät nach wenigstens einem der Ansprüche 1 bis 10, wobei der wenigstens eine Entladewiderstand (R4, R5, R6, R7, R8, R9) einen Widerstandswert zwischen 47 kΩ und 120 kΩ hat.

## Claims

1. Medical device with a power adaptor for connection to an AC power supply, wherein the power adaptor has a rectifier and at least one filter capacitor (C1) for smoothing the DC voltage to be outputted from the power adaptor, as well as at least one discharge resistor (R4, R5, R6, R7, R8, R9) for discharging the filter capacitor (C1),
wherein the power adaptor has a circuit which is electrically connected to the discharge resistor (R4, R5, R6, R7, R8, R9) and the filter capacitor (C1) and which is configured to detect a switched-on state of the medical device, in which an input AC voltage is applied to the power adaptor, across the entire input voltage range and to switch the discharge resistor (R4, R5, R6, R7, R8, R9) in parallel to the filter capacitor (C1) only when the circuit does not detect a switched-on state of the medical device, wherein the circuit has a first transistor (T2) for connecting the discharge resistor (R4, R5, R6, R7, R8, R9) in parallel with the filter capacitor (C1), which is arranged and dimensioned such that it switches through and effects a discharging of the filter capacitor (C1) when a control capacitor (C4) connected to the base or the gate of the transistor (T2) is sufficiently charged, wherein the control capacitor (C4) is connected to a control transistor (T1) which is arranged and dimensioned such that the control transistor (T1) regularly switches through and discharges the control capacitor (C4) as long as the power adaptor is supplied with line voltage.

2. Medical device according to claim 1, wherein
the first transistor (T2) is a field effect transistor (T2).

3. Medical device according to at least one of claims 1 or 2, wherein
the rectifier has a rectifier bridge and the circuit is configured to collect a voltage between an input-side connection of the rectifier bridge and an output-side connection of the rectifier bridge and to detect a supply line voltage applied to the input-side connection of the rectifier bridge.

4. Medical device according to claim 3, wherein
the circuit has a voltage divider connected between an input of the rectifier bridge and the negative output of the rectifier bridge and a first capacitor (C3), connected in parallel to a resistor (R2) of the voltage divider, which in the case of an application of a supply line voltage is charged to a voltage defined by the voltage divider.

5. Medical device according to at least one of claims 1 to 4, wherein
the base-emitter path of the control transistor (T1) is connected in parallel to the resistor of the voltage divider and the control transistor (T1) switches through in the range from >50 V_{Ac} ... 264 V_{AC} in the case of an application of a supply line voltage.

6. Medical device according to at least one of claims 1 to 5, wherein
the control transistor (T1) is a bipolar transistor.

7. Medical device according to at least one of claims 2 to 6, wherein
a second capacitor (C4) is switched in parallel to a gate-drain path of the field effect transistor (T2), which second capacitor, in its charged state, has the effect that the field effect transistor (T2) switches through such that the at least one discharge resistor (R4, R5, R6, R7, R8, R9) is connected in parallel to the at least one filter capacitor (C1).

8. Medical device according to claims 6 and 7, wherein
the second capacitor (C4) is connected in parallel to a collector-emitter path of the control transistor (T1), such that the second capacitor (C4) is discharged when the control transistor (T1) is switched through, such that the at least one discharge resistor (R4, R5, R6, R7, R8, R9) is separated from the at least one filter capacitor (C1) when a supply line voltage is applied to the input of the rectifier.

9. Medical device according to at least one of claims 1 to 8, wherein
the circuit has at least one Zener diode (D1; D2) for limiting voltage.

10. Medical device according to at least one of claims 1 to 9, wherein
the circuit has several discharge resistors (R4, R5, R6, R7, R8, R9) connected in parallel to one another.

11. Medical device according to at least one of claims 1 to 10, wherein
the at least one discharge resistor (R4, R5, R6, R7, R8, R9) has a resistance value of between 47 kΩ and 120 kΩ.

## Revendications

1. Appareil médical avec un bloc d'alimentation destiné à être raccordé à un réseau à courant alternatif, dans lequel le bloc d'alimentation présente un redresseur et au moins un condensateur de filtrage (C1) pour lisser une tension continue devant être délivrée par le bloc d'alimentation ainsi qu'au moins une résistance de décharge (R4, R5, R6, R7, R8, R9) pour décharger le condensateur de filtrage (C1),
dans lequel
le bloc d'alimentation présente un circuit, qui est relié électriquement à la résistance de décharge (R4, R5, R6, R7, R8, R9) et au condensateur de filtrage (C1) et qui est réalisé pour détecter un état allumé de l'appareil médical, dans lequel une tension alternative d'entrée est appliquée sur le bloc d'alimentation, sur la totalité de la zone de tension d'entrée et pour brancher la résistance de décharge (R4, R5, R6, R7, R8, R9) seulement alors de manière parallèle au condensateur de filtrage (C1) lorsque le circuit ne détecte aucun état allumé de l'appareil médical, dans lequel le circuit présente un premier transistor (T2) pour brancher en parallèle la résistance de décharge (R4, R5, R6, R7, R8, R9) par rapport au condensateur de filtrage (C1) qui est disposé et est dimensionné de telle sorte qu'il conduit et qu'il provoque une décharge du condensateur de filtrage (C1) lorsqu'un condensateur de commande (C4) relié à la base ou à la grille du transistor (T2) est suffisamment chargé, dans lequel le condensateur de commande (C4) est relié à un transistor de commande (T1), qui est disposé et dimensionné de telle sorte que le transistor de commande (T1) conduit régulièrement et décharge le condensateur de commande (C4) jusqu'à ce que le bloc d'alimentation soit alimenté en tension de réseau.

2. Appareil médical selon la revendication 1, dans lequel le premier transistor (T2) est un transistor à effet de champ (T2).

3. Appareil médical selon au moins l'une quelconque des revendications 1 ou 2, dans lequel le redresseur présente un pont redresseur et le circuit est configuré pour prélever une tension entre une borne côté entrée du pont redresseur et une borne côté sortie du point redresseur et pour détecter une tension de réseau d'alimentation appliquée sur la borne côté entrée du pont redresseur.

4. Appareil médical selon la revendication 3, dans lequel le circuit présente un diviseur de tension branché entre une entrée du pont redresseur et la sortie négative du pont redresseur et un premier condensateur (C3) branché en parallèle par rapport à une résistance (R2) du diviseur de tension, qui est chargé dans le cas d'une application d'une tension de réseau d'alimentation sur une tension définie par le diviseur de tension.

5. Appareil médical selon au moins l'une quelconque des revendications 1 à 4, dans lequel le tronçon émetteur de base du transistor de commande (T1) est branché en parallèle par rapport à la résistance du diviseur de tension et le transistor de commande (T1) conduit dans le cas d'une application d'une tension de réseau d'alimentation dans la plage > 50 V_{AC} ... 264 V_{AC}.

6. Appareil médical selon au moins l'une quelconque des revendications 1 à 5, dans lequel le transistor de commande (T1) est un transistor bipolaire.

7. Appareil médical selon au moins l'une quelconque des revendications 2 à 6, dans lequel un deuxième condensateur (C4) est branché en parallèle par rapport à un tronçon grille-drain du transistor à effet de champ (T2), lequel a pour effet dans son état chargé que le transistor à effet de champ (T2) conduit si bien que l'au moins une résistance de décharge (R4, R5, R6, R7, R8, R9) est branchée en parallèle par rapport à l'au moins un condensateur de filtrage (C1).

8. Appareil médical selon les revendications 6 et 7, dans lequel le deuxième condensateur (C4) est branché en parallèle par rapport à un tronçon émetteur collecteur du transistor de commande (T1), de sorte que le deuxième condensateur (C4) est déchargé lorsque le transistor de commande (T1) conduit de sorte que l'au moins une résistance de décharge (R4, R5, R6, R7, R8, R9) est séparée de l'au moins un condensateur de filtrage (C1) lorsqu'une tension de réseau d'alimentation est appliquée sur l'entrée du redresseur.

9. Appareil médical selon au moins l'une quelconque des revendications 1 à 8, dans lequel le circuit présente au moins une diode Zener (D1 ; D2) pour limiter la tension.

10. Appareil médical selon au moins l'une quelconque des revendications 1 à 9, dans lequel le circuit présente plusieurs résistances de décharge (R4, R5, R6, R7, R8, R9) branchées en parallèle les unes par rapport aux autres.

11. Appareil médical selon au moins l'une quelconque des revendications 1 à 10, dans lequel l'au moins une résistance de décharge (R4, R5, R6, R7, R8, R9) a une valeur de résistance entre 47 kΩ et 120 kΩ.
